(19) Europäisches Patentamt
European Patent Office
Office européen des brevets

(11) EP 1 258 533 A1

(12) EUROPEAN PATENT APPLICATION

(43) Date of publication:
**20.11.2002 Bulletin 2002/47**

(51) Int Cl.[7]: **C12Q 1/42**, G01N 27/30

(21) Application number: **01830304.0**

(22) Date of filing: **11.05.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Tecnav S.N.C. di Marco Antonio Stracuzzi E C.**
**00139 Roma (IT)**

(72) Inventors:
- **Botre', Claudio**
  **00185 Roma (IT)**
- **Botre', Francesco**
  **00185 Roma (IT)**
- **Mazzei, Francesco**
  **00176 Roma (IT)**
- **Podesta', Elisabetta**
  **00191 Roma (IT)**

(74) Representative: **Tansini, Elio Fabrizio**
**C/O Bugnion S.p.A.,**
**Viale Lancetti, 17**
**20158 Milano (IT)**

# (54) Process for detection and determination of toxic agents and related biosensor

(57) The present invention relates to a method of detection and determination of toxic agents, characterized by the following steps: reacting at least one substrate with an enzymatic system comprising the FAc and FAl enzymes with formation of at least one product P; measuring the concentration of said at least one product P; causing an interaction of the enzymatic system with at least one toxic agent included in the compounds having an organo-phosphoric and/or organo-halogenated structure, present in the matrix submitted to analysis, by addition to said at least one substrate and to the enzymatic system of a sample to be analysed; measuring the concentration reduction in said at least one product P by detection of a signal of an electric nature. In addition, the present invention relates to a bi- or tri-enzymatic biosensor.

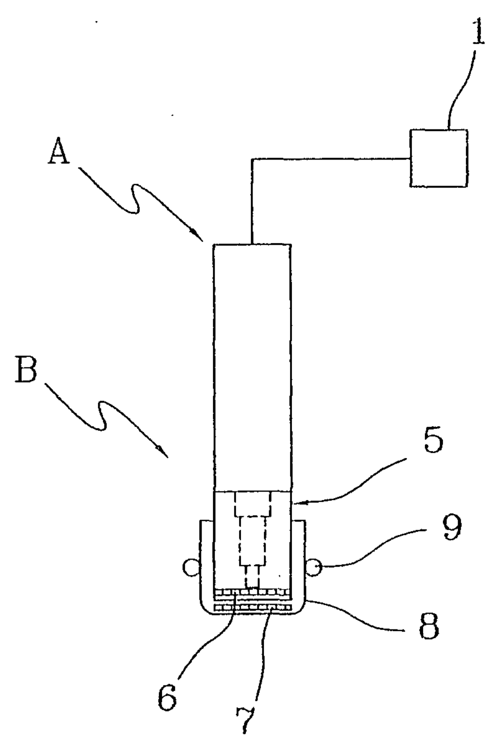


FIG. 2

EP 1 258 533 A1

## Description

The present invention relates to a process for detection and determination of toxic agents. In particular, the present invention relates to a process for detection and determination of toxic agents belonging to the class of compounds having an organo-phosphoric and organo-halogenated structure, as well as to a related biosensor.

It is known that analytic processes commonly employed for detection of toxic agents, generally consist of successive stages: drawing of samples, transportation of same to appropriate analysis laboratories, screening and/or monitoring on such samples and, lastly, a final analysis for confirmation, with "classical" instrumental methods for identification of all toxic components present therein that, from screening and/or monitoring, give positive or uncertain results.

An ideal screening and/or monitoring should ensure detection of all toxic agents belonging to one or more toxicological classes and provided with dangerous effects for the living organisms, without the risk of false negative values.

The great variety of structures belonging to such different classes of toxic agents, as well as the requirement of carrying out analytic determinations *in situ*, prevent from resorting to the above mentioned common analysis methods. Said methods in fact are very arduous and require very sophisticated and expensive apparatus such as liquid chromatography associated with mass spectroscopy (LC-MS), gas chromatography associated with mass spectroscopy (GC-MS), etc.

Taking into consideration their technical and construction features, these apparatus are not suitable for detection of toxic agents, due both to operating and logistic reasons, represented by long periods of time for carrying out analyses, the impossibility of moving the apparatus from the laboratories in which they are, the requirement of qualified staff for execution of the different analytic operations, which will, consequently, bring about very high costs.

Even high performance liquid chromatography is surely a very valid method, but on carrying it out very long and arduous preliminary operations are required such as pre-concentrations and derivatization reactions that, among other things, call for the availability of updated files for comparison of the different intermediates and internal standards obtained in the different analytic operations.

Classical bioanalytic methods such as ELISA and other immunologic methods are not always able to give reliable results and fall again into the above mentioned time and cost problems.

In addition, not to be forgotten is the fact that under emergency situations detection of toxic agents must be as quick as possible. Quickness in detection, in this case, becomes a decisive and essential factor since damage associated with the toxic agent which is often lethal sometimes expresses itself in very short periods of time.

It should be pointed out that each class of structures under examination comprises many chemical species that, with "classical" analysis methods, must be determined individually and in a specific manner.

In this connection it is reminded that in the case of foodstuff contamination by complex molecular structures provided with toxicity, such as algal toxins, the international scientific community imposes application of toxicity tests on animals which are expensive, long and bloody.

As an alternative to classical analysis methods described up to this point, particular biosensors have been proposed which are able to detect constituents belonging to different classes of toxic agents.

A biosensor is a measurement device based on coupling of a physico-chemical transduction system of an obtained signal with a biological detection system of a particular interaction between a biological system (enzyme) and a particular chemical species (toxic agent).

The number of biosensors that can be obtained is very wide due to the high number of biologically active molecules that can be incorporated in such analysis devices and due to the wide selection that can be done in the field of true sensors.

It is known that some biosensors show sensitiveness and selectivity towards some chemical compounds until fractions of parts per billion (ppb), even under experimental conditions far from balance, with methods that are not invasive or restrictive for the sensor, provided with great versatility in carrying out measurements in a continuous manner.

In addition, biosensors enable important responses to be obtained in very short periods of time.

In fact the response time is one of the fundamental features in particular application fields and absolutely essential in many practical applications.

There are analytic methods based on biosensors which are able to convert into electrically measurable signals, variations in the concentration of a compound obtained from a reaction catalysed by an enzyme which is irreversibly-inhibited by organo-phosphoric compounds.

Such analytic methods are based on inhibition of the acetylcholinesterase enzyme carried out by organo-phosphoric compounds.

A first drawback that can be found in a biosensor utilizing the acetylcholinesterase enzyme is due to the fact that the catalytic activity of the enzyme is irreversibly inhibited as a result of the interaction with the organo-phosphoric

compound (toxic agent).

In addition, the number of the analyses to be carried out with the above mentioned type of biosensor (employing the acetylcholinesterase enzyme) is not very high and can be evaluated in a limited number of analyses and requires a regeneration treatment between a determination and the subsequent one.

Another drawback of the biosensor utilizing the acetylcholinesterase enzyme resides in that the enzyme can be employed for organo-phosphoric compounds but not for organo-chlorine compounds.

In the case herein described the envisaged biosensor is made up of:

- a biological system represented by a mono- or poly-enzymatic system; and
- a physico-chemical system, represented by an electrochemical measurement system.

Document EP 723 020 A describes a method of determining organo-phosphoric pesticides in a matrix of vegetable origin by use of a biosensor utilizing the acid phosphatase (FAc) enzyme and the glucose-oxidase (GOD) enzyme in the presence of a glucose-6-phosphate (G6P) substrate.

The method described in document EP 723 020 A1 is based on reversible inhibition of FAc due to the action of organo-phosphoric pesticides.

Therefore, there is a need for a method of detecting and determining organo-halogenated and organo-phosphoric compounds possibly present in any matrix submitted to analysis, which is quick, easy to be carried out and provided with high sensitiveness.

Availability of a biosensor is required which can be employed without any loss of sensitiveness for a high number of determinations, and is suitable for detection and determination of toxic organo-halogenated and organo-phosphoric compounds possibly present in a generic matrix submitted to analysis.

The technical problem to be solved therefore consists in obtaining a method and a biosensor suitable for detection and determination of toxic agents without the drawbacks of known techniques of the above mentioned type and relating to a method based on use of acetylcholinesterase.

In particular, the technical problem to be solved consists in making available a method and the related biosensor for detection and determination of organo-halogenated and organo-phosphoric compounds utilizing a reversible interaction between the enzyme and the organo-halogenated or organo-phosphoric compounds possibly present in the matrix submitted to analysis.

The solution to said problem is suggested by the Applicant for the present patent application that has found a method of detection and determination, by a suitable biosensor, of compounds having organo-halogenated and organo-phosphoric structures, present in different matrices.

The method being the object of the present invention comprises the following steps:

- reacting at least one substrate selected from the group consisting of: glucose-6-phosphate, ascorbate-2-phosphate, phenyl-phosphate, p-hydroxyphenyl-phosphate, p-aminophenyl-phosphate, N-nitrophenyl-phosphate, 1-naphthyl-phosphate, 3-indoxyl-phosphate with an enzymatic system comprising the acid phosphatase enzyme (FAc) and the alkaline phosphatase enzyme (FAl) with formation of at least one product P, obtained from reaction between the enzymes and said at least one substrate;
- measuring the concentration of said at least one product P obtained from reaction between the enzymatic system and said at least one substrate;
- obtaining a reversible interaction of the enzymatic system with at least one toxic agent comprising the compounds of an organo-phosphoric and organo-halogenated structure, present in the matrix submitted to analysis, by addition to the enzymatic system of at least one substrate and the sample to be analysed; and
- determining the concentration reduction in said at least one product P obtained from the reaction between the enzymatic system and said at least one substrate, by detection of a signal of an electric nature.

Generally the organo-phosphoric and organo-halogenated compounds belong to the classes of the pesticides (herbicides, bactericides, fungicides, insecticides, rat poisons) and the chemical agents (nerve gases, etc).

The matrices submitted to analysis for detection and determination of toxic agents can therefore be of different nature and can be in different phases: the aeriform phase such as for example the atmospheric air; the liquid phase such as for example surface or deep water bodies; the solid phase such as for example the soil and manufactured articles of different nature.

In the case of a toxic agent present in a solid matrix it is necessary to bring the toxic agent into a solution resorting to appropriate washing, with hydrophilic or hydrophobic solutions possibly containing surface-active agents of anionic, cationic or non-ionic nature, for example. The above mentioned toxic agents interact with the enzymatic system made up of FAc and/or FAl and/or GOD, by a reversible interaction (enzyme-toxic agent).

From interaction between the toxic agent and enzymatic system, taking place without an important degradation of the enzymes, a signal of electric nature is determined.

Contrary to that which happens with other types of enzymes, in the case of the present invention the interaction does not impair the enzyme activity that stay almost unchanged, while there is an inactivation of the structure of the toxic agents that, in opposition to enzymes, are degraded.

The catalytic action can be obtained in two different pH ranges: an acid pH for the FAc enzyme and an alkaline pH for the FAl enzyme.

Both enzymes, FAc and FAl, interact in a reversible manner with the organo-phosphoric compounds, whereas the 2,4-D(2,4-dichlorophenoxyacetic acid) interacts in a reversible manner with the FAl alone and does not interact with the FAc.

The method in accordance with the invention will be now described in more detail with reference to the accompanying drawings, given by way of non-limiting example, in which:

- Fig. 1 is a diagrammatic representation of the amperometric sensor (electrode) of the electrochemical biosensor;
- Fig. 2 is a diagrammatic representation of the electrochemical biosensor;
- Fig. 3 is a diagrammatic representation of an integrated biosensor-capacity cell system;
- Fig. 4 is a diagrammatic representation of an apparatus suitable for practical accomplishment of the method of the present invention;
- Fig. 5 is a representation of the trend of the current signal recorded during the interaction of the enzymatic system with a toxic agent. These graphs are obtained with the bi-enzymatic FAc/FAl or tri-enzymatic FAc/FAl/GOD biosensors. On the x-coordinate the time in minutes is reproduced and on the y-coordinate the intensity of the electric current signal expressed in nanoampere (nA) is reproduced;
- Figs. 6 and 7 reproduce reaction examples of some substrates with the respective enzymes and the values of the discharge potentials of the related products.

With reference to Fig. 1, an amperometric sensor A is used to manufacture the device being the object of the present invention. Sensor A is able to detect the hydrogen peroxide, or a product P, depending on whether a tri-enzymatic or a bi-enzymatic biosensor are respectively used.

Within the context of the present invention the hydrogen peroxide too is considered as a generic product P.

Sensor A is for example connected to a unit 1 comprising an amperometre/potentiostat (not shown in the drawings), and is made up of a sensor body 2, a cathode 3, preferably of Ag/AgCl, an anode 4, an unloosable (i.e. susceptible of being unscrewed) cap 5 and a membrane 6.

Membrane 6 can be of a type permeable to the hydrogen peroxide or to a product P, depending on the type of enzymatic configuration used. For instance, membrane 6 may consist of cellulose acetate.

Detection and determination of toxic agents can be carried out following two embodiments, both of them being the object of the present invention.

A first embodiment is represented by a tri-enzymatic biosensor, as reproduced in Figs. 2 and 3 for example.

With reference to Fig. 2, a biosensor B is shown. Biosensor B is made up of sensor A and a plurality of membranes 7 and 8. The outermost membrane 8 is a membrane for dialysis. Preferably the outer membrane 8 is made of cellulose acetate.

In the tri-enzymatic biosensor embodiment the outer membrane 8 contains the FAc and FAl enzymes immobilized therein.

In the bi-enzymatic biosensor embodiment the outer membrane 8 does not contain the enzymes immobilized therein.

Preferably, the intermediate membrane 7 is made of nylon with carboxylic, amino or other functional groups on its surface. For instance, the intermediate membrane 7 is a membrane made of nylon 6 or 6.6.

In the tri-enzymatic biosensor embodiment the intermediate membrane 7 contains the GOD enzyme immobilized therein.

In the bi-enzymatic biosensor embodiment the intermediate membrane 7 contains the FAc and FAl enzymes immobilized therein.

Preferably, the inner membrane 6 of cap 5 is a membrane made of cellulose acetate in the case of a tri-enzymatic system or it is a dialysis membrane in the case of a bi-enzymatic system.

Membranes 7 and 8 are maintained joined to each other by a locking ring 9 engaging them on the unloosable cap 7 of sensor A.

With reference to Fig. 3, an integrated system C is shown for direct measurement of the toxic agents present in an aeriform or liquid matrix. The integrated system C is made up of a biosensor B of the above described type and a cell D. Identified by E is the membrane 6 of sensor A and the plurality of membranes 7 and 8 of biosensor B.

Advantageously, the tri-enzymatic biosensor shown in Figs. 2 and 3 employs an enzymatic system comprising the FAc and FAl enzymes and a third enzyme such as for example the GOD (glucose-oxidase) enzyme.

The GOD enzyme catalyses the following reaction:

$$\text{Glucose} + O_2 \overset{\text{GOD}}{\rightleftarrows} \text{glucono-lactone} + H_2O_2$$

In this first embodiment use of three enzymes is provided: FAc, FAl and GOD and the glucose-6-phosphate substrate.

$$G6P + H_2O \overset{\text{FAc}}{\rightleftarrows} \text{glucose} + H_2PO_4 \qquad (1)$$

$$G6P + H_2O \overset{\text{FAl}}{\rightleftarrows} \text{glucose} + H_2PO_4 \qquad (2)$$

$$\text{Glucose} + O_2 \overset{\text{GOD}}{\rightleftarrows} \text{glucono-lactone} + H_2O_2 \qquad (3)$$

Concentration of $H_2O_2$ (a generic product P obtained from the glucose-6-phosphate substrate) is recorded by an amperometric sensor A consisting of a specific electrode for detection of hydrogen peroxide.

The reactions taking place at the electrodes are the following:

$$H_2O_2 \text{ -----> } O_2 + 2H^+ + 2e^-$$

$$1/2O_2 + 2H^+ + 2e^- \text{ ----> } H_2O$$

Variation in the current intensity is correlated with variations in the concentration of the produced hydrogen peroxide.

The glucose required for carrying out reaction (3) comes from the hydrolysis reactions (1) and (2) catalysed by the two phosphatases.

In the embodiment of the tri-enzymatic biosensor, as shown in Figs. 2 and 3, and in the embodiment of the bi-enzymatic biosensor, as shown in Figs. 2 and 3, the FAc and FAl enzymes can be both immobilized on one alone of said membranes 7 and 8.

For instance, in the case of the tri-enzymatic biosensor the outer membrane 8 contains both FAc/FAl enzymes. For instance, in the case of the bi-enzymatic biosensor the intermediate membrane 7 contains both FAc/FAl enzymes.

Alternatively, the FAc can be immobilized on a membrane and the FAl can be immobilized on another membrane distinct from the preceding one.

Advantageously, an embodiment providing for the two phosphatases, FAc and FAl, to be immobilized on one of said membranes is preferred.

The presence of toxic agents in solution, capable of reversibly interacting with the phosphatase activities, causes a reduction in the glucose production as reproduced in reactions (1) and (2).

The reduction in the glucose concentration involves detection of a lower concentration of $H_2O_2$.

In this way a calibration curve with standard solutions of toxic agent can be constructed, versus the progressively detected values of $H_2O_2$ concentrations.

A second embodiment being the object of the present invention is represented by a bi-enzymatic biosensor as reproduced in Figs. 2 and 3, for example.

Advantageously, the Applicant has found it useful to determine the concentration of a toxic agent of an organophosphoric or organo-halogenated structure, through employment of at least one substrate of the above stated type. The substrates, following the hydrolysis reaction caused by acid and alkaline phosphatases, give rise to at least one

product P.

With the bi-enzymatic sensor the products P, obtained from the reaction between the enzymatic system and the substrate therein employed, are able to directly discharge to the work electrode, depending on the values of the potential differences applied to the electrodes.

The hydrolysis reactions of some substrates are depicted in Figs. 6 and 7.

For example, the ascorbate-2-phosphate reaction is the following:

$$\text{Ascorbate-2-phosphate} + H_2O \quad \xrightleftharpoons{FAc+FAl} \quad \text{Ascorbate} + H_2PO_4^-$$

The ascorbate formed from the reaction is discharged to the (platinum) anode, following the oxidation process.

Advantageously, organo-phosphoric pesticides provided with great toxicity such as Malathion, Methyl-Parathion and Paraoxon for example, are determined by means of the biosensor being the object of the present invention both by physico-chemical immobilization of the enzymes in a purified form on an appropriate membrane and by means of the acid phosphatase contained in a thin layer of a vegetable tissue drawn from an organism containing it, from potato (Solanum tuberosum) for example.

The inside of the hydrogen peroxide-based electrode is filled with a solution of 0.01 M KCl and 0.01 M $K_2HPO_4$/ $KH_2PO_4$ (pH = 6.6).

A first function of the outer membrane 9 is to inhibit possible release of enzymes into the matrix under examination and to prevent possible interactions between the enzymes and other components, possibly present in the sample, of a microbial or viral nature for example.

Should these interactions occur, they could lead to drawbacks such as inactivation of the biocatalytic material.

Preferably, the phosphatases, FAc and FAl, are immobilized on a nylon membrane with functional groups present on its surface.

The "active" part of the biosensor, made up of the portions identified in the diagram by numbers 7 and 8, is fastened to the end of a "cap" which is screwed down to the "fixed" part of sensor A.

The biosensor, when not in use, is maintained in a solution of 0.1 M citrate buffer at pH=6.0.

During the inactivity periods it is sufficient to disassemble the cap containing the bio-catalytic membrane and keep it dry in a freezer at a temperature ranging between -15°C and -20°C.

In the case of the tri-enzymatic biosensor, calibration is carried out by a series of amperometric measurements for which a constant electric potential difference is applied which is included between +650 mV and +700 mV between a platinum anode (work electrode) and an Ag/AgCl cathode (reference electrode). The work electrode and the reference electrode constitute the electrochemical detection system of the biosensor.

Measurements are carried out following two different operating modalities:

- the first modality involves use of the biosensor immersed in the sample; and
- the second modality involves use of the biosensor in an overturned position as shown in Fig. 3.

In a first case 0.5 ml of a 0.1 M citrate buffer at pH=6.0 and 0.2 mM $MgCl_2$ (as a FAl cofactor) were employed. The citrate buffer and $MgCl_2$ were disposed in a cell preferably made of glass at a constant temperature thermostatically controlled to 25 ± 0.1°C for example, under constant magnetic stirring, as reproduced in Fig. 4.

With reference to Fig. 4, the biosensor B is inserted in a thermostatically controlled cell 10 the temperature of which is constantly controlled by thermostat 11. Cell 10 contains a magnetic armature 12 and a buffer in which the biosensor B is partly immersed. A magnetic stirrer 13 ensures mixing of the solution. Biosensor B is connected to a unit 1, represented by an amperometre/potentiostat. Unit 1 is connected to a graphic recording unit 14 or other data acquisition system. Unit 1 can transmit the signal, through a connecting network, to a remote position 15.

The pH value of the buffer employed in measurement represents the most favourable condition to obtain the maximum result in the catalytic response of the three enzymes: FAc, FAl and GOD enzymes.

Subsequently, predetermined proportional amounts of a standard solution of G6P were gradually added at time intervals of two minutes, the corresponding variations in the intensity values of the electric current being recorded.

Preferably, the G6P concentration obtained in the measurement buffer must be included between 0.5 and 0.8 millimole.

The intensity values of the electric current were recorded using a measurement system depicted in Fig. 4.

In a second case a capacity cell integrated with the biosensor was employed, as reproduced in Fig. 3.

Measurements were carried out putting 0.2 mL of a 0.1 M citrate buffer solution at pH=6.0 and 0.2 mM $MgCl_2$ in cell D, adding a known volume of the standard substrate solution and recording, in this case too, the corresponding

variations in the intensity values of the electric current.

Fig. 3 is a diagrammatic representation of the integrated biosensor-cell (C) system, for direct measurement of toxic agents.

In the case of a tri-enzymatic sensor, sensor A is an electrochemical sensor adapted to measure the hydrogen peroxide originating from substrate G-6-P.

In the case of a bi-enzymatic sensor, A is an electrochemical sensor suitable to measure the products P obtained from the reaction between the enzymatic system and a substrate selected from the group comprising: ascorbate-2-phosphate, phenylphosphate, p-hydroxyphenyl phosphate, p-aminophenyl phosphate, p-nitrophenyl phosphate, 1-naphthylphosphate, 3-indoxylphosphate.

The products P are able to directly discharge to the work electrode (anode) following an oxidation process, for given values of differences in the applied potential, see Figs. 6 and 7.

The following example is given for the purpose of illustrating, in a non-limiting sense, detection and determination of a toxic agent, the Paraoxon, by the method being the object of the present invention.

Determination is carried out following the scheme reproduced in Fig. 4, in which the biosensor is of the type shown in Figs. 2 and 3. In the capacity cell 11 a substrate to a given concentration is added. Selection of this concentration results from the requirement of obtaining a good response by the sensor towards the substrate (which is generally intermediate between the maximum and minimum values of the calibration curve obtained for the substrate) and therefore enhancing the system sensitiveness towards the interaction reaction with the toxic agent.

As regards determination of the toxic agent, after stabilization of the current signal following addition of the substrate, known proportional and increasing amounts of the toxic agent (a compound having an organo-phosphoric and/or organo-halogenated structure, herein Paraoxon) are added under magnetic stirring and, after a preestablished time interval, a lowering in the current intensity is noticed which is due to interaction of the toxic agent with the FAc and FAl and thus to a reduction in the concentration of the substance produced following hydrolysis by the enzymes.

In this way it was possible to construct specific calibration curves for the different organo-phosphoric and organo-halogenated toxic agents.

Fig. 5 shows the trend of the current signal recorded during a typical experiment for detection of a toxic agent with the previously characterized biosensor.

The first arrow, reproduced in Fig. 5, refers to addition of substrate S; the next arrow refers to addition of the toxic agent I marking the beginning of the interaction process with the enzymatic system, which results in lowering of the current intensity.

The tri-enzymatic biosensor employed following the scheme depicted in Fig. 4, shows a linearity range included between 1-20 ppb towards Paraoxon, after a 20 minutes' incubation. The biosensor was re-utilised for about 150-180 determinations without any treatment.

The bi-enzymatic biosensor employed following the scheme depicted in Fig. 3 shows a linearity range included between 0.5 and 20 ppb towards Paraoxon, after a 20 minutes' incubation. In this case the substrate utilised was ascorbate-2-phosphate. The biosensor was re-utilised for about 150-180 determinations without any treatment.

The tri-enzymatic biosensor employed in accordance with the modalities depicted in Fig. 3 shows the performance results to be compared with those obtained with the same biosensor employed following the scheme in Fig. 4.

Advantageously, determination of the electric current intensity can be carried out by a voltametric technique.

Another possibility for detection and consequent determination of the concentration of toxic agents is to resort to a voltametric analysis technique associated with the enzymatic hydrolysis reaction enabling detection of the specific "discharges" of the different chemical species P generated following the enzymatic reactions.

The possible substrates to be employed for this type of determination are: ascorbate-2-phosphate, phenylphosphate, p-hydroxyphenyl phosphate, p-aminophenyl phosphate, p-nitrophenyl phosphate, 1-naphthylphosphate, 3-indoxylphosphate.

The voltametric technique involves use of three electrodes:

- a work electrode, on the surface of which the reactions of the electro active species take place and which is made up of inert materials such as: platinum (Pt), gold (Au), glassy carbon, carbon paste and other metals to be used individually or in appropriate combinations;
- a reference electrode entrusted with the task of controlling the potential of the work electrode;
- a counter-electrode (or auxiliary electrode) which is passed through by a flux of current from the work electrode and generated by the discharge of the electro active species on the surface thereof. This electrode, of inert material, is generally made up of platinum.

The method being the object of the present invention enables toxic agents to be detected in the surrounding environment (air, water, soil) by an appropriate poly-enzymatic biosensor in very short periods of time, with rather simple apparatus and very reduced costs.

The sensitiveness of the detection method is very high and can even reach fractions of parts per billion (ppb).
In addition, in accordance with the principle of operation of the biosensor based on the interaction between the toxic agent and the enzymatic system, application of such principle also extends to other classes of toxic agents, such as algal toxins and other substances.
For instance, the okadaic acid, a very toxic substance was detected by this method in concentrations lower than 0.5 parts per billion.
Advantageously, the method is based on the interaction between the enzyme (or enzymes) and the toxic agent, with a consequent generation of a signal of the electric nature which is simultaneously sent to the detection system operating *in situ* and/or connected with a network and to a remote position 15 as shown in Fig. 4.

## Claims

1. A method of detection and determination of toxic agents **characterized in that** it comprises the following steps:

   - reacting at least one substrate selected from the group consisting of: glucose-6-phosphate, ascorbate-2-phosphate, phenyl-phosphate, p-hydroxyphenyl-phosphate, p-aminophenyl-phosphate, N-nitrophenyl-phosphate, 1-naphthyl-phosphate, 3-indoxyl-phosphate with an enzymatic system comprising an FAc enzyme and an FAl enzyme with formation of at least one product P, obtained from the reaction between the enzymes and said at least one substrate;
   - measuring the concentration of said at least one product P obtained from the reaction between the enzymatic system and said at least one substrate;
   - causing a reversible interaction between the enzymatic system and at least one toxic agent selected from the toxic agents having an organo-phosphoric and/or organo-halogenated structure, present in the matrix submitted to analysis, by addition to said at least one substrate and to the enzymatic system of a sample to be analysed; and
   - measuring the concentration reduction in said at least one product P obtained from the reaction between the enzymatic system and said at least one toxic agent, by detection of a signal of an electric nature.

2. The method as claimed in claim 1, wherein the substrate is glucose-6-phosphate and the enzymatic system is made of the acid phosphatase FAc enzyme, the alkaline phosphatase FAl enzyme and the glucose oxidase GOD enzyme.

3. The method as claimed in claim 2, wherein the FAc enzyme and the FAl enzyme are immobilized on a single outer membrane and the GOD enzyme is immobilized on another intermediate membrane.

4. The method as claimed in claim 3, wherein the outer membrane is a dialysis membrane.

5. The method as claimed in claim 3, wherein the intermediate membrane is a membrane made of nylon having functional groups on its surface.

6. The method as claimed in claim 2, wherein the FAc enzyme is immobilized on one membrane and the FAl enzyme is immobilized on another membrane distinct from the preceding one.

7. The method as claimed in claim 1, wherein the substrate is selected from ascorbate-2-phosphate, phenylphosphate, p-hydroxyphenyl phosphate, p-aminophenyl phosphate, p-nitrophenyl phosphate, 1-naphthylphosphate, 3-indoxylphosphate and the enzymatic system comprises the FAc enzyme and the FAl enzyme.

8. The method as claimed in claim 5, wherein the FAc enzyme and the FAl enzyme are immobilized on a single intermediate membrane.

9. The method as claimed in claim 8, wherein the intermediate membrane is a membrane made of nylon having functional groups, carboxylic and amino groups for example, on its surface.

10. The method as claimed in claim 8, wherein the FAc enzyme is immobilized on one membrane and the FAl enzyme is immobilized on another membrane distinct from the preceding one.

11. The method as claimed in claim 1, wherein the FAc enzyme is contained in a thin layer of vegetable tissue.

**12.** A biosensor (B) for detection and determination of toxic agents in a matrix to be analysed comprising: a sensor (A) comprising a cathode (3), an anode (4) and a permeable membrane (6) disposed externally of said sensor; said biosensor (B) is **characterized by** an outer membrane (8) for dialysis, placed close to said membrane (6), and an intermediate membrane (7) containing at least one enzyme belonging to the FAc, FAl and GOD group and interposed between said membrane (6) and said membrane (8).

**13.** The biosensor (B) as claimed in claim 12, wherein on the intermediate membrane thereof the GOD enzyme is immobilized and on the outer membrane the FAc and FAl enzymes are immobilized.

**14.** The biosensor (B) as claimed in claim 12, wherein on the intermediate membrane thereof the FAc and FAl enzymes are immobilized.

**15.** The biosensor (B) as claimed in claim 12, wherein the membrane (6) is a membrane of cellulose acetate.

**16.** The biosensor (B) as claimed in claim 12, wherein the membrane (7) is a membrane of nylon having functional groups, carboxylic and amino groups for example, on its surface.

**17.** The biosensor (B) as claimed in claim 12, wherein the FAc enzyme is contained in a thin layer of vegetable tissue.

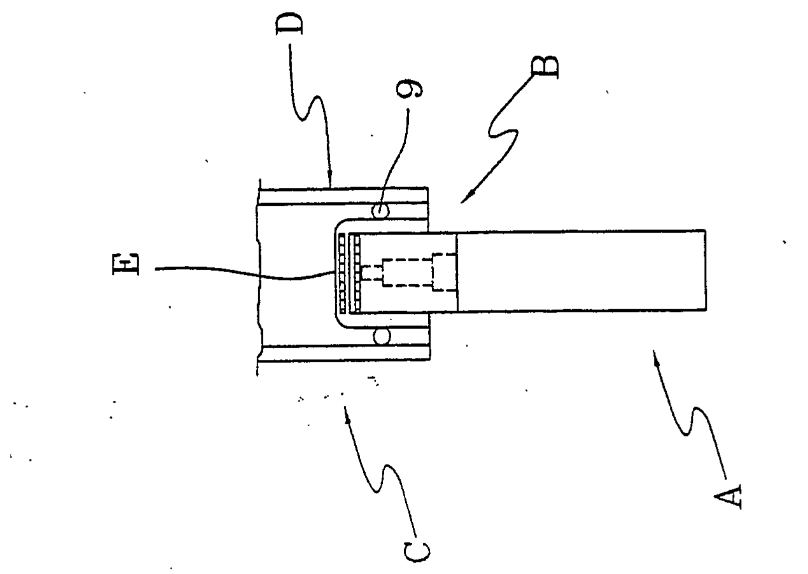


FIG. 3

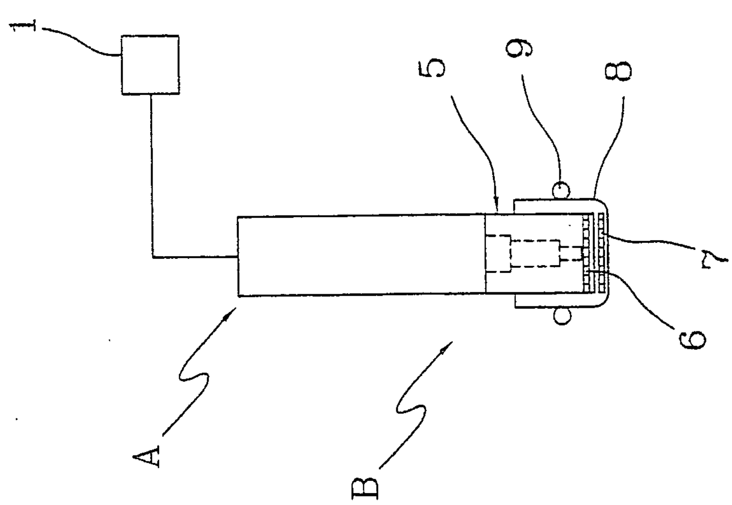


FIG. 2

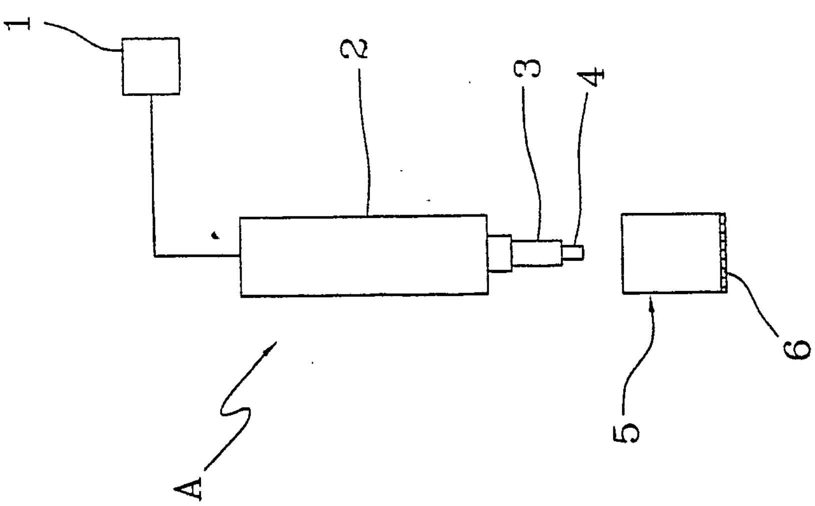


FIG. 1

FIG. 4

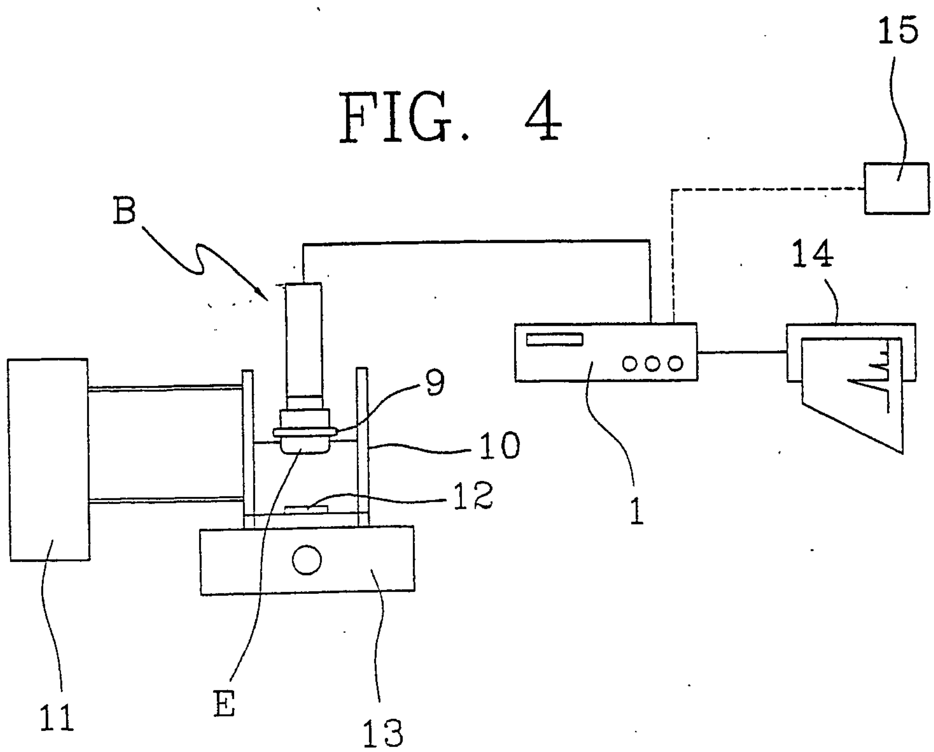


FIG. 5

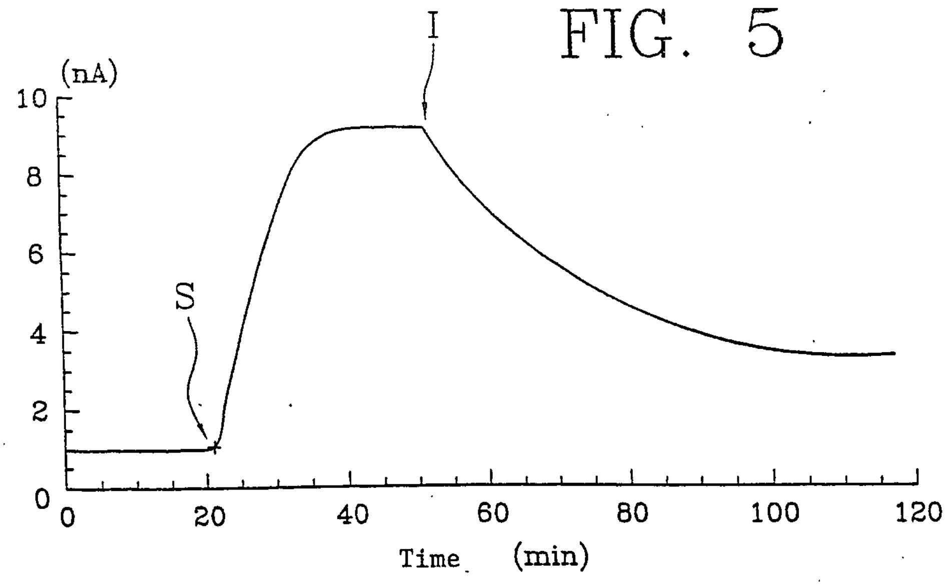

FIG. 6

phenyl-phosphate + $H_2O \overset{FAc+FAl}{\rightleftharpoons}$ phenol + $H_2PO_4^-$

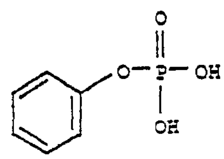

phenyl-phosphate

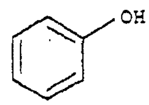

phenol

determination : +0.7V (vs Ag/AgCl)

p-aminophenyl-phosphate + $H_2O \overset{FAc+FAl}{\rightleftharpoons}$ 4-p-aminophenol + $H_2PO_4^-$

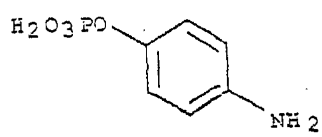

4-p-aminophenyl-phosphate

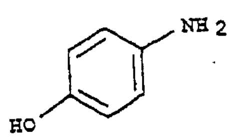

4-p-aminophenol

determination : +0.1V (vs Ag/AgCl)

FIG.7

3-indoxyl phosphate $+ H_2O \xrightarrow{FAc+FAl} $ indigo $+ H_2PO_4^-$

indigo =*3H-Indol-3-one, 2-(1,3-dihydro-3-oxo-2H-indol-2-ylidene)-1,2-dihydro- (9CI)*

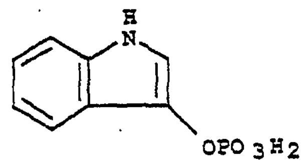

3-indoxyl phosphate

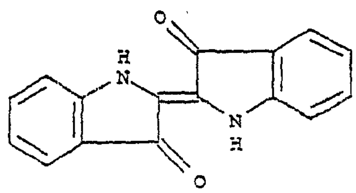

indigo

Determination: two reversible processes -0.4V e +0.3V (vs Ag/AgCl)

1-naphthyl-phosphate $+ H_2O \xrightarrow{FAc+FAl}$ 1-naphthol $+ H_2PO_4^-$

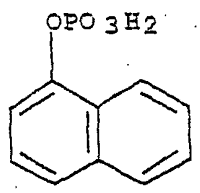

1-naphthyl-phosphate

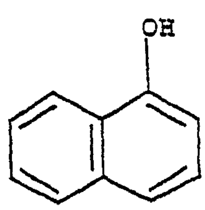

1-naphthol

determination + 0.4V (vs Ag/AgCl)

European Patent Office

# EUROPEAN SEARCH REPORT

Application Number
EP 01 83 0304

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X<br><br>Y | EP 0 723 020 A (BARILLA FLLI G & R) 24 July 1996 (1996-07-24)<br>* page 2, line 55 - page 4, line 6; claims 14-17 *<br>* page 5, line 44 - line 46 * | 12,15-17<br><br>1-11,13, 14 | C12Q1/42<br>G01N27/30 |
| Y | US 5 846 753 A (TRIPATHY SUKANT K ET AL) 8 December 1998 (1998-12-08)<br>* column 6, line 34 - column 8, line 14 *<br>* column 7, line 54 - line 58; figure 3 * | 1-11,13, 14 | |
| A | WO 90 05910 A (I STAT CORP) 31 May 1990 (1990-05-31)<br>* page 64, line 3 - line 12 *<br>* page 18, line 24 - page 20, line 3 *<br>* page 37, line 12 - page 38, line 16 *<br>* page 86, line 17 - line 24; table II *<br>* page 96, line 1 - page 97, line 21 * | 1-17 | |
| A | NISTOR C ET AL: "Bioanalytical tools for monitoring polar pollutants" WASTE MANAGEMENT, ELSEVIER, NEW YORK, NY, US, vol. 19, no. 2, April 1999 (1999-04), pages 147-170, XP004163914 ISSN: 0956-053X<br>* page 158, column 2, paragraph 5; table 4 * | 1-17 | TECHNICAL FIELDS SEARCHED (Int.Cl.7)<br>C12Q<br>G01N |
| A | EP 0 424 586 A (IMMUNOSENS SPA) 2 May 1991 (1991-05-02)<br>* abstract *<br>* page 2, line 24 *<br>* page 6, line 43-45 * | 1-17 | |

The present search report has been drawn up for all claims

| Place of search | Date of completion of the search | Examiner |
|---|---|---|
| MUNICH | 8 October 2001 | Tilkorn, A-C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 83 0304

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2001

| Patent document cited in search report | | Publication date | Patent family member(s) | | | Publication date |
|---|---|---|---|---|---|---|
| EP 0723020 | A | 24-07-1996 | IT | MI950109 | A1 | 23-07-1996 |
| | | | BR | 9600178 | A | 07-10-1997 |
| | | | EP | 0723020 | A1 | 24-07-1996 |
| | | | PL | 311091 | A1 | 05-08-1996 |
| | | | TR | 960796 | A2 | 21-10-1996 |
| US 5846753 | A | 08-12-1998 | NONE | | | |
| WO 9005910 | A | 31-05-1990 | CA | 2002848 | A1 | 14-05-1990 |
| | | | CA | 2221178 | A1 | 14-05-1990 |
| | | | EP | 0442969 | A1 | 28-08-1991 |
| | | | JP | 3137612 | B2 | 26-02-2001 |
| | | | JP | 2000065791 | A | 03-03-2000 |
| | | | JP | 4503249 | T | 11-06-1992 |
| | | | JP | 3105919 | B2 | 06-11-2000 |
| | | | KR | 175917 | B1 | 15-05-1999 |
| | | | SG | 45431 | A1 | 16-01-1998 |
| | | | WO | 9005910 | A1 | 31-05-1990 |
| | | | US | 5554339 | A | 10-09-1996 |
| | | | US | 5200051 | A | 06-04-1993 |
| | | | US | 5837446 | A | 17-11-1998 |
| | | | US | 5837454 | A | 17-11-1998 |
| | | | US | 5063081 | A | 05-11-1991 |
| | | | US | 5212050 | A | 18-05-1993 |
| | | | US | 5466575 | A | 14-11-1995 |
| EP 0424586 | A | 02-05-1991 | AU | 643465 | B2 | 18-11-1993 |
| | | | AU | 6497390 | A | 02-05-1991 |
| | | | BR | 9005440 | A | 17-09-1991 |
| | | | CA | 2028609 | A1 | 27-04-1991 |
| | | | EP | 0424586 | A2 | 02-05-1991 |
| | | | JP | 3216547 | A | 24-09-1991 |
| | | | ZA | 9008547 | A | 31-07-1991 |

EPO FORM P0459

For more details about this annex : see Official Journal of the European Patent Office, No. 12/82